# FASCICULE DE BREVET EUROPEEN

(11) **EP 0 662 081 B1**
(45) Date de publication et mention de la délivrance du brevet: **12.03.1997**
(21) Numéro de dépôt: 93919418.9
(22) Date de dépôt: 06.09.1993
(51) Int. Cl.: C07K 5/06, A61K 38/05

(54) **DERIVES D'UREIDO-ACETAMIDE, LEUR PREPARATION ET LES MEDICAMENTS LES CONTENANT**
UREIDOACETAMID-DERIVATE,IRHE HERSTELLUNG UND SIE ENTHALTENDE MEDIKAMENTE
UREIDO-ACETAMIDE DERIVATIVES, PREPARATION THEREOF AND DRUGS CONTAINING SAME

(30) Priorité: 11.09.1992 FR 9210838
(43) Date de publication de la demande: 12.07.1995
(73) Titulaire: RHONE-POULENC RORER S.A., 92160 Antony (FR)
(72) Inventeur: DUBROEUCQ, Marie-Christine, F-95880 Enghien-les-Bains (FR); GUYON, Claude, F-94100 Saint-Maur-des-Fossés (FR)
(74) Mandataire: Savina, Jacques
(86) Numéro de dépôt international: FR9300847
(87) Numéro de publication internationale: WO9406825

(56) Documents cités:
- WO-A-91/12264

## Description

Dans les demandes de brevet WO 91/12264 et WO 91/13907 sont décrits des dérivés d'uréido-acétamide utiles comme antagonistes de la cholécystokinine (CCK) et de la gastrine.

Il a maintenant été trouvé que les composés de formule : 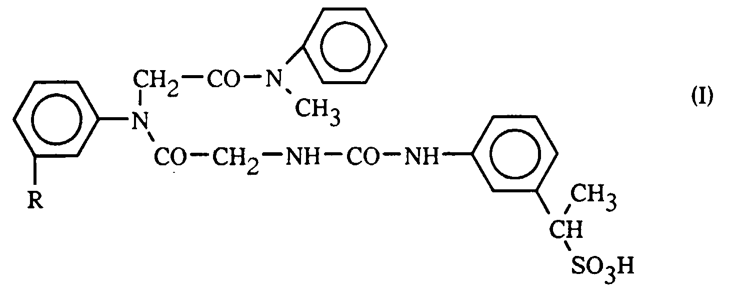 dans laquelle R représente un atome d'hydrogène ou un radical méthoxy, leurs sels, leurs racémiques et leurs énantiomères présentent de façon inattendue des propriétés antagonistes de la CCK très supérieures à celles des uréidoacétamides des demandes de brevet WO 91/12264 et WO 91/13907.

La présente invention a donc pour objet les composés de formule (I), leurs sels, leurs énantiomères, leur préparation et les médicaments les contenant.

Les composés de formule (I) peuvent être préparés par action d'un dérivé de formule : 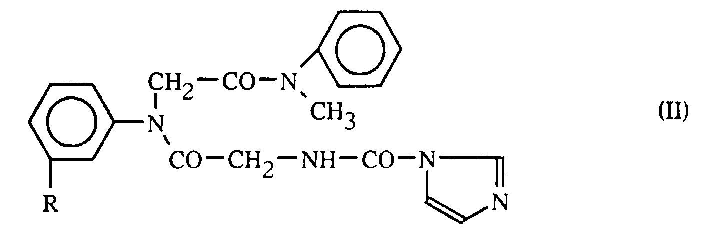 dans laquelle R représente un atome d'hydrogène ou un radical méthoxy, sur une amine de formule : 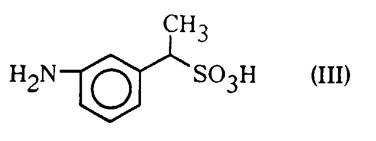 sous forme d'un sel.

Cette réaction s'effectue généralement au sein d'un solvant inerte, tel que le benzène ou le toluène, à la température d'ébullition du milieu réactionnel. Comme sels peuvent être utilisés les sels de tétraalkylammonium ou de trialkylphénylammonium et de préférence de tétra-n-butyl ammonium.

Les dérivés de formule (II) peuvent être obtenus par application ou adaptation des méthodes décrites dans les demandes de brevet WO 91/12264 et WO 91/13907.

Les énantiomères peuvent être préparés à partir des précurseurs chiraux du composé de formule (III).

De préférence, les énantiomères sont préparés à partir d'un sel de tétraalkyl ammonium (Forme B) de l'amine de formule (III) ou du sel d'hydroquinine (Forme A) de l'amine de formule (III).

Les composés de formule (I) peuvent être purifiés par les méthodes connues habituelles, par exemple, par cristallisation, précipitation, chromatographie ou extraction.

Les composés de formule (I) et leurs énantiomères peuvent être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'un acide organique sur un composé de formule (I) ou ses énantiomères, dans un solvant.

Comme exemples de sels pharmaceutiquement acceptables, peuvent être cités les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthylglucamine).

Les composés de formule (I) et leurs énantiomères présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement de la prévention des psychoses, des troubles anxieux, des attaques de panique, de la maladie de Parkinson, de la diskynésie tardive, du syndrôme du colon irritable, de la pancréatite aiguë, des ulcères, des désordres de la motilité intestinale, de certaines tumeurs sensibles à la CCK, comme régulateur de l'appétit, dans le sevrage aux traitements chroniques et abus d'alcool ou de médicaments et comme constricteur de la pupille de l'oeil.

Ces composés ont également un effet de potentialisation sur l'activité analgésique des médicaments narcotiques et non narcotiques. En outre, ils peuvent avoir un effet analgésique propre.

Par ailleurs, les composés ayant une forte affinité pour les récepteurs CCK modifient les capacités de mémorisation. En conséquence, ces composés peuvent être efficaces dans les troubles de la mémoire.

L'affinité des composés de formule (I) pour les récepteurs CCK a été déterminée selon une technique inspirée de celle de A. SAITO et Coll. (J. Neuro. Chem., 37, 483-490 (1981) au niveau du cortex cérébral.

Dans ce test, la CI₅₀ des composés de formule (I) est inférieure à 2 nM.

Par ailleurs, il est connu que les produits qui reconnaissent les récepteurs centraux de la CCK ont une spécificité similaire pour les récepteurs de la gastrine dans le tractus gastrointestinal (BOCK et Coll., J. Med. Chem., 32, 16-23 (1989) ; REYFELD et Coll., Am. J. Physiol., 240, G 255-266 (1981) ; BEINFELD et Coll., Neuropeptides, 3, 411-427 (1983)).

Les composés de formule (I) présentent une faible toxicité. Leur DL₅₀ est supérieure à 40 mg/kg par voie sous cutanée chez les souris.

D'un intérêt particulier sont les composés suivants :
- acide {{[N-(méthoxy-3 phényl) N-(N-méthyl N-phényl-carbamoylméthyl) carbamoylméthyl]-3 uréido}-3 phényl}-1 éthanesulfonique-(RS)
- acide (-)-{{[N-(méthoxy-3 phényl) N-(N-méthyl N-phényl-carbamoylméthyl) carbamoylméthyl]-3 uréido}-3 phényl}-1 éthanesulfonique
- acide {{[N-(N-méthyl N-phényl-carbamoylméthyl) N-phényl-carbamoylméthyl]-3 uréido}-3 phényl}-1 éthanesulfonique-(RS).

Les exemples suivants illustrent l'invention sans la limiter.

### EXEMPLE 1

A une solution de 5,5 g d'{[(imidazolyl-1) carboxamido]-2 N-(méthoxy-3 phényl) acétamido}-2 N-méthyl N-phényl-acétamide dans 120 cm³ de toluène, on ajoute 6,7 g d'(amino-3 phényl)-1 éthanesulfonate de tétra-n-butyl ammonium-(RS). Le mélange réactionnel est agité à reflux pendant 5 heures, puis concentré à sec sous pression réduite (2,7 kPa) à 45°C. Le résidu est dissous dans 200 cm³ de chlorure de méthylène et la solution obtenue est lavée par 200 cm³ d'une solution aqueuse d'acide chlorhydrique 2N, puis par 2 fois 200 cm³ d'eau. La phase organique est séchée sur sulfate de magnésium et concentrée à sec sous pression réduite (2,7 kPa) à 40°C. Le produit brut obtenu est agité 30 minutes dans 100 cm³ d'oxyde de diisopropyle. Le produit insoluble est séparé par filtration puis dissous dans 20 cm³ d'acétone. On ajoute 3,0 g de nonafluorobutanesulfonate de potassium en solution dans 10 cm³ d'acétone puis 25 cm³ d'oxyde de diisopropyle. Le produit insoluble est séparé par filtration, lavé par 2 fois 5 cm³ d'acétone puis 4 fois 15 cm³ d'oxyde de diisopropyle et séché à l'air. On obtient ainsi 5,5 g de {{[N-(méthoxy-3 phényl) N-(N-méthyl N-phényl-carbamoylméthyl) carbamoylméthyl]-3 uréido}-3 phényl}-1 éthanesulfonate de potassium -(RS) fondant à environ 180°C.
Spectre de R.M.N.: (300 MHz; DMSO d6) :
δ(ppm):

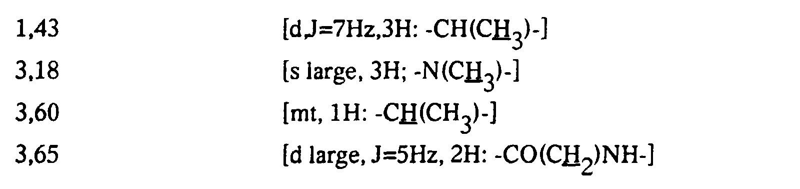

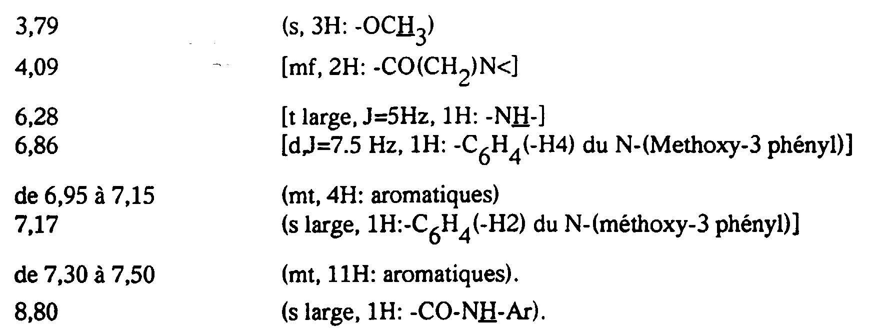

L'(amino-3 phényl)-1 éthanesulfonate de tétra-n-butyl ammonium-(RS), peut être préparé de la manière suivante : à une solution de 17,8 g de (nitro-3 phényl)-1 éthanesulfonate de tétra-n-butyl ammonium-(RS) dans 200 cm³ d'éthanol, on ajoute 0,8 g de palladium sur noir à 5%. La suspension est agitée pendant 3 heures à une température voisine de 25°C sous atmosphère d'hydrogène (100 kPa). Le catalyseur est séparé par filtration et le filtrat est concentré à sec sous pression réduite (2,7 kPa) à 40°C. On obtient ainsi 16,7 g d'(amino-3 phényl)-1 éthanesulfonate de tétra-n-butyl ammonium-(RS) sous forme d'une huile utilisée telle quelle dans les synthèses ultérieures.

Le (nitro-3 phényl)-1 éthanesulfonate de tétra-n-butyl ammonium-(RS) peut être préparé de la manière suivante : à une solution de 20,8 g de sulfite de sodium dans 260 cm³ d'eau on ajoute 25,3 g de (bromo-1 éthyl)-1 nitro-3 benzène-(RS). Le mélange réactionnel est agité à 80°C pendant 5 heures, refroidi à environ 25°C et coulé dans 2,5 litres d'une solution aqueuse de dihydrogénophosphate de potassium 0,5 M. On ajoute 40 g d'hydrogénosulfate de tétra-n-butyl ammonium. Le mélange est extrait par 3 fois 500 cm³ de chlorure de méthylène. Les phases organiques réunies sont lavées par 2 fois 500 cm³ d'eau, séchées sur sulfate de magnésium et concentrées à sec sous pression réduite (2,7 kPa) à 40°C. On obtient ainsi 51,4 g de (nitro-3 phényl)-1 éthanesulfonate de tétra-n-butyl ammonium-(RS), sous forme d'une huile utilisée telle quelle dans les synthèses ultérieures.

Le (bromo-1 éthyl)-1 nitro-3 benzène-(RS) peut être préparé selon la méthode décrite par E. Felder et coll., J. Med. Chem., 13, 559 (1970).

L'{[(imidazolyl-1) carboxamido]-2 N-(méthoxy-3 phényl) acétamido}-2 N-méthyl N-phényl-acétamide peut être préparé de la manière suivante : à une solution de 3,0 g de N,N'-diimidazolecarbonyle dans 30 cm³ de tétrahydrofuranne anhydre, on ajoute une solution de 3,1 g d'[amino-2 N-(méthoxy-3 phényl) acétamido]-2 N-méthyl N-phényl-acétamide dans 30 cm³ de tétrahydrofuranne anhydre. La solution est agitée pendant 16 heures à une température voisine de 25°C puis concentrée à sec sous pression réduite (2,7 kPa) à 40°C. Le résidu est dissous dans 50 cm³ d'acétate d'éthyle et la solution obtenue est lavée par 4 fois 30 cm³ d'eau. La phase organique est séchée sur sulfate de magnésium puis concentrée à sec sous pression réduite (2,7 kPa) à 4°C. On obtient, après recristallisation dans l'acétate d'éthyle, 3,5 g d'{[(imidazolyl-1) carboxamido]-2 N-(méthoxy-3 phényl) acétamido}-2 N-méthyl N-phényl-acétamide fondant à 146°C

L'[amino-2 N-(méthoxy-3 phényl) acétamido]-2 N-méthyl N-phényl-acétamide peut être préparé de la manière suivante : à une solution de 5,5 g de [N-(méthoxy-3 phényl) phtalimido-2 acétamido]-2 N-méthyl N-phényl-acétamide dans 60 cm³ de méthanol, on ajoute 1,3 g d'hydrate d'hydrazine. Le mélange réactionnel est agité à reflux pendant 30 minutes puis, après refroidissement, on ajoute 100 cm³ d'eau. Le mélange est concentré à environ 100 cm³ puis amené à pH 9 avec une solution aqueuse de soude 2N et extrait par 2 fois 50 cm³ d'acétate d'éthyle. Les phases organiques réunies sont lavées par 2 fois 50 cm³ d'eau, séchées sur sulfate de magnésium puis concentrées à sec sous pression réduite (2,7 kPa) à 40°C. On obtient ainsi 3,0 g d'[amino-2 N-(méthoxy-3 phényl) acétamido]-2 N-méthyl N-phényl-acétamide sous forme d'une huile utilisée telle quelle dans les synthèses ultérieures.

Le [N-(méthoxy-3 phényl) phtalimido-2 acétamido]-2 N-méthyl N-phényl-acétamide peut être préparé de la manière suivante : à une suspension de 80,6 g d'acide [N-(méthoxy-3 phényl) phtalimido-2 acétamido]-2 acétique dans 900 cm³ de dichloro-1,2 éthane on ajoute 10 cm³ de diméthylformamide puis en 1 heure, 30,2 g de dichlorure d'oxalyle. Le mélange est agité 2 heures à une température voisine de 25°C, puis on ajoute 58,6 g de N-méthyl aniline en 45 minutes. Le mélange réactionnel est agité 2 heures à une température voisine de 25°C, lavé par 2 fois 500 cm³ d'eau puis 300 cm³ d'une solution aqueuse saturée d'hydrogénocarbonate de sodium, séché sur sulfate de magnésium et concentré à sec sous pression réduite (2,7kPa) à 40°C. Le résidu est agité une heure dans 300 cm³ d'oxyde de diisopropyle, le produit insoluble est séparé par filtration, lavé par 3 fois 60 cm³ d'oxyde de diisopropyle et séché à l'air. On obtient ainsi, 84 g de [N-(méthoxy-3 phényl) phtalimido-2 acétamido]-2 N-méthyl N-phényl-acétamide fondant à 137°C.

L'acide [N-(méthoxy-3 phényl) phtalimido-2 acétamido]-2 acétique peut être préparé de la manière suivante : à une solution de 42,0 g de [N-(méthoxy-3 phényl) phtalimido-2 acétamido]-2 acétate de tert-butyle dans 500 cm³ de chlorure de méthylène, on ajoute 74,0 g d'acide trifluoroacétique. La solution obtenue est agitée à reflux pendant 5 heures, puis concentrée à sec sous pression réduite (2,7 kPa) à 40°C. Le résidu est agité pendant une heure dans 100 cm³ d'oxyde de diisopropyle, le produit insoluble est séparé par filtration, lavé par 3 fois 40 cm³ d'oxyde de diisopropyle et séché à l'air. On obtient ainsi, 36 g d'acide [N-(méthoxy-3 phényl) phtalimido-2 acétamido]-2 acétique fondant à 203°C.

Le [N-(méthoxy-3 phényl) phtalimido-2 acétamido]-2 acétate de tert-butyle peut être préparé de la manière suivante : à une solution de 96 g de N-(méthoxy-3 phényl) phtalimido-2 acétamide dans 1000 cm³ de tétrahydrofuranne anhydre, on ajoute en 30 minutes 14,9 g d'une suspension huileuse (60% en poids) d'hydrure de sodium. La suspension est agitée pendant 4 heures à une température voisine de 20°C puis on ajoute en 15 minutes 72,7 g de bromoacétate de tert-butyle. Le mélange réactionnel est agité pendant 16 heures à une température voisine de 25°C, hydrolysé lentement par 50 cm³ d'eau, puis concentré à sec sous pression réduite. Le résidu obtenu est agité pendant une heure dans 400 cm³ d'eau, le produit insoluble est séparé par filtration, lavé par 3 fois 100 cm³ d'eau, 2 fois 100 cm³ d'oxyde de diisopropyle et séché à l'air. On obtient ainsi, 82,0 g de [N-(méthoxy-3 phényl) phtalimido-2 acétamido]-2 acétate de tert-butyle fondant à 148°C.

Le N-(méthoxy-3 phényl) phtalimido-2 acétamide peut être préparé de la manière suivante : à une solution de 26,0 g de méthoxy-3 aniline dans 200 cm³ de chlorure de méthylène, on ajoute 22,0 g de triéthylamine puis 48,0 g de chlorure de phtalimido-2 acétyle en solution dans 300 cm³ de chlorure de méthylène en maintenant la température à environ 20°C. Le mélange réactionnel est agité pendant 4 heures, à cette température, puis on ajoute 800 cm³ d'eau. Le produit insoluble est séparé par filtration, lavé par 3 fois 100 cm³ d'eau et séché à l'air. On obtient ainsi 65,0 g de N-(méthoxy-3 phényl) phtalimido-2 acétamide fondant à 171°C.

Le chlorure de phtalimido-2 acétyle peut être préparé selon la méthode décrite par W. GRASSMANN et E. SCHULTE-UEBBING, Chem. Ber., 83, 244-247, (1950).

### EXEMPLE 2

A une solution de 2,1 g d'{[(imidazolyl-1) carboxamido]-2 N-(méthoxy-3 phényl) acétamido)-2 N-méthyl N-phényl-acétamide dans 130 cm³ de toluène, on ajoute 2,7 g d'(amino-3 phényl)-1 éthanesulfonate de tétra-n-butyl ammonium, forme B. Le mélange réactionnel est agité à reflux pendant 5 heures, puis concentré à sec sous pression réduite (2,7 kPa) à 45°C. Le résidu est dissous dans 100 cm³ de chlorure de méthylène et la solution obtenue est lavée par 50 cm³ d'une solution aqueuse d'acide chlorhydrique 2N, puis par 2 fois 50 cm³ d'eau. La phase organique est séchée sur sulfate de magnésium et concentrée à sec sous pression réduite (2,7 kPa) à 40°C. Le produit brut obtenu est agité 30 minutes dans 50 cm³ d'oxyde de diisopropyle. Le produit insoluble est séparé par filtration puis dissous dans 6 cm³ d'acétone. On ajoute 1,2 g de nonafluorobutanesulfonate de potassium en solution dans 3 cm³ d'acétone puis 5 cm³ d'oxyde de diisopropyle. La gomme insoluble est séparée, puis agitée pendant 1 heure dans 12 cm³ d'un mélange d'acétone et d'oxyde de diisopropyle (50/50 en volumes). Le produit insoluble est séparé par filtration, lavé par 2 fois 5 cm³ d'un mélange d'acétone et d'oxyde de diisopropyle (50/50 en volumes) puis 4 fois 5 cm³ d'oxyde de diisopropyle et séché à l'air. On obtient ainsi 1,55 g de (-)-{{[N-(méthoxy-3 phényl) N-(N-méthyl N-phényl-carbamoylméthyl) carbamoylméthyl]-3 uréido}-3 phényl}-1 éthanesulfonate de potassium fondant à environ 180°C.
[α]_{D}²⁰ = - 5,0° ± 0,5 (C = 0,888 %; Méthanol).
Spectre de R.M.N.: (300 MHz; DMSO d6: forme (-))
d(ppm):

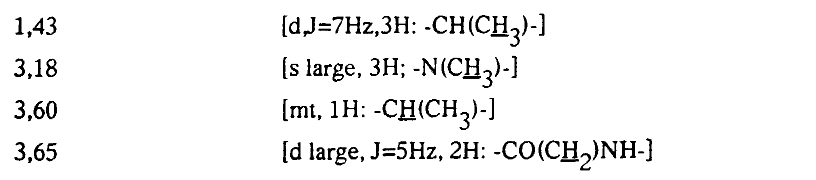

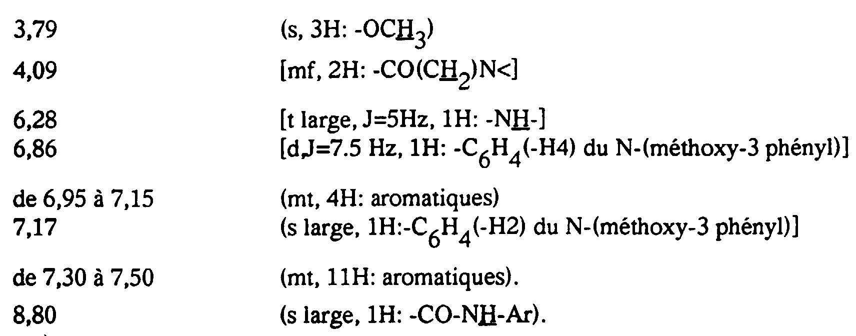

L'(amino-3 phényl)-1 éthanesulfonate de tétra-n-butyl ammonium, forme B, peut être préparé de la manière suivante : à une solution de 2,8 g de (nitro-3 phényl)-1 éthanesulfonate de tétra-n-butyl ammonium, forme B, dans 50 cm³ d'éthanol, on ajoute 0,2 g de palladium sur noir à 5 %. La suspension est agitée pendant 2 heures à une température voisine de 25°C sous atmosphère d'hydrogène (100 kPa). Le catalyseur est séparé par filtration et le filtrat est concentré à sec sous pression réduite (2,7 kPa) à 40°C. On obtient ainsi 2,8 g d'(amino-3 phényl)-1 éthanesulfonate de tétra-n-butyl ammonium, forme B, sous forme d'une huile utilisée telle quelle dans les synthèses ultérieures.

Le (nitro-3 phényl)-1 éthanesulfonate de tétra-n-butyl ammonium, forme B, peut être préparé de la manière suivante : à une solution de 10,5 g de (nitro-3 phényl)-1 éthanesulfonate de N-benzylquininium, mélange des formes A et B (environ 15/85 en moles), dans 16 cm³ d'acétone, on ajoute 5,2 g de nonafluorobutanesulfonate de potassium en solution dans 12 cm³ d'acétone. Le produit insoluble est séparé par filtration, puis dissous dans 9 cm³ d'eau. On ajoute 8,4 cm³ d'une solution aqueuse d'acide chlorhydrique 1N et 1,15 g de R-(-)-phénylglycinol. La solution obtenue est concentrée à sec sous pression réduite (2,7 kPa) à 50°C. Le résidu obtenu est extrait par 3 fois 15 cm³ d'acétonitrile à reflux. Les phases organiques sont réunies, et concentrées à environ 7 cm³ ; après refroidissement, les cristaux sont séparés par filtration et dissous dans 7,5 cm³ d'une solution aqueuse de soude 1N. La solution obtenue est lavée par 8 fois 25 cm³ d'oxyde de diéthyle, puis on ajoute 60 cm³ d'une solution aqueuse de dihydrogénophosphate de potassium 0,5 M et 2,3 g d'hydrogénosulfate de tétra-n-butyl ammonium. Le mélange est extrait par 3 fois 30 cm³ de chlorure de méthylène. Les phases organiques réunies sont séchées sur sulfate de magnésium et concentrées à sec sous pression réduite (2,7 kPa) à 40°C. On obtient ainsi 2,8 g de (nitro-3 phényl)-1 éthanesulfonate de tétra-n-butyl ammonium, forme B, sous forme d'une huile utilisée telle quelle dans les synthèses ultérieures.

Le (nitro-3 phényl)-1 éthanesulfonate de N-benzylquininium, mélange des formes A et B, peut être préparé de la manière suivante : à une solution de 17,2 g de (nitro-3 phényl)-1 éthanesulfonate de potassium-(RS) dans 400 cm³ d'eau, on ajoute 87 g de dihydrogénophosphate de potassium et 32,4 g de chlorure de N-benzyl quininium. Le mélange est extrait par 2 fois 300 cm³ de chlorure de méthylène. Les phases organiques réunies sont lavées par 2 fois 200 cm³ d'eau, séchées sur sulfate de magnésium et concentrées à sec sous pression résuite (2,7 kPa) à 40°C. La meringue obtenue est dissoute dans 120 cm³ de propanol-2 à reflux. Après refroidissement, les cristaux sont séparés par filtration et lavés par 2 fois 15 cm³ de propanol-2. On obtient, après 2 recristallisations dans 350 puis 500 cm³ de propanol-2, 15,6 g de (nitro-3 phényl)-1 éthanesulfonate de N-benzylquininium, forme A, fondant à environ 110°C. [α]_{D}²⁰ = - 151,3° ± 1,5 (C = 1,009 % ; Méthanol). Les solutions propanoliques sont réunies et concentrées à sec sous pression réduite (2,7 kPa) à 45°C. On obtient ainsi 25,0 g de (nitro-3 phényl)-1 éthanesulfonate de N-benzylquininium, mélange des formes A et B (environ 15/85 en moles).

Le (nitro-3 phényl)-1 éthanesulfonate de potassium-(RS) peut être préparé de la manière suivante : à une solution de 20,8 g de sulfite de sodium dans 260 cm³ d'eau on ajoute 25, 3 g de (bromo-1 éthyl)-1 nitro-3 benzène-(RS). Le mélange réactionnel est agité à 80°C pendant 5 heures, refroidi à environ 25°C et coulé dans 2,5 litres d'une solution aqueuse de dihydrogénophosphate de potassium 0,5 M. On ajoute 40 g d'hydrogénosulfate de tétra-n-butyl ammonium. Le mélange est extrait par 3 fois 500 cm³ de chlorure de méthylène. Les phases organiques sont lavées par 2 fois 500 cm³ d'eau, séchées sur sulfate de magnésium concentrées à sec sous pression réduite (2,7 kPa) à 40°C. L'huile obtenue est dissoute dans 65 cm³ d'acétone et on ajoute 34 g de nonafluorobutanesulfonate de potassium en solution dans 75 cm³ d'acétone. Le produit insoluble est séparé par filtration, lavé par 3 fois 50 cm³ d'oxyde de diisopropyle et séché à l'air. On obtient ainsi 22,4 g de (nitro-3 phényl)-1 éthanesulfonate de potassium-(RS), fondant à une température supérieure à 260°C et utilisé tel quel dans les synthèses ultérieures.

Le (bromo-1 éthyl)-1 nitro-3 benzène-(RS) peut être préparé selon la méthode décrite par E. FELDER et Coll., J. Med. Chem., 13, 559 (1970).

### EXEMPLE 3

A une solution de 2,8 g d'{(imidazolyl-1) carboxamido]-2 N-phénylacétamido]-2 N-méthyl N-phényl-acétamide dans 70 cm³ de toluène, on ajoute 6,7 g d'(amino-3 phényl)-1 éthanesulfonate de tétra-n-butyl ammonium-(RS). Le mélange réactionnel est agité à reflux pendant 5 heures, puis concentré à sec sous pression réduite (2,7 kPa) à 45°C. Le résidu est dissous dans 100 cm³ de chlorure de méthylène et la solution obtenue est lavée par 100 cm³ d'une solution aqueuse d'acide chlorhydrique 2N, puis par 2 fois 100 cm³ d'eau. La phase organique est séchée sur sulfate de magnésium et concentrée à sec sous pression réduite (2,7 kPa) à 40°C. Le produit brut obtenu est agité 30 minutes dans 60 cm³ d'oxyde de diisopropyle. Le produit insoluble est séparé par filtration puis dissous dans 20 cm³ d'acétone. On ajoute 1,9 g de nonafluorobutanesulfonate de potassium en solution dans 10 cm³ d'acétone. Le produit insoluble est séparé par filtration, lavé par 4 fois 5 cm³ d'acétone puis 4 fois 8 cm³ d'oxyde de diisopropyle et séché à l'air. On obtient ainsi 5,5 g de ([(N-(N-méthyl N-phényl-carbamoylméthyl) N-phényl-carbamoylméthyl]-3 uréido)-3 phényl}-1 éthanesulfonate de potassium-(RS) fondant à environ 210°C.

L'{[(imidazolyl-1) carboxamido}-2 N-phényl-acétamido}-2 N-méthyl N-phényl-acétamide peut être préparé de la manière suivante : à une solution de 31 g de N,N'-diimidazole carbonyle dans 300 cm³ de tétrahydrofuranne anhydre, on ajoute une solution de 37 g d'(amino-2 N-phényl-acétamido)-2 N-méthyl-acétamide dans 150 cm³ de tétrahydrofuranne anhydre. La solution est agitée pendant 3 heures à une température voisine de 25°C, puis concentrée à sec sous pression réduite (2,7 kPa) à 40°C. Le résidu est dissous dans 500 cm³ d'acétate d'éthyle et la solution obtenue est lavée successivement par 4 fois 300 cm³ d'eau distillée et par 300 cm³ d'une solution aqueuse saturée de chlorure de sodium. La phase organique est séchée sur sulfate de magnésium puis concentrée à sec sous pression réduite (2,7 kPa) à 40°C. On obtient après recristallisation dans l'acétate d'éthyle 33,3 g d'{[(imidazolyl-1) carboxamido}-2 N-phényl-acétamido}-2 N-méthyl N-phényl-acétamide fondant à 120°C.

L'(amino-2 N-phényl-acétamido)-2 N-méthyl N-phényl-acétamide peut être préparé de la manière suivante : à une solution de 1,4 g de N-méthyl N-phényl (N-phényl phtalimido-2 acétamido)-2 acétamide dans 15 cm³ de méthanol, on ajoute 0,25 g d'hydrate d'hydrazine. Le mélange réactionnel est agité à reflux pendant 2 heures. Après refroidissement et addition de 5 cm³ d'une solution aqueuse 4N d'acide chlorhydrique, le produit insoluble est séparé par filtration. Le filtrat est concentré à sec sous pression réduite (2,7 kPa) ) 30°C. Le résidu obtenu est dissous dans 10 cm³ d'eau distillée, puis la solution obtenue est lavée par 10 cm³ d'éther diéthylique, alcanisée avec 0,5 g de soude en pastilles et extraite par 2 fois 20 cm³ d'acétate d'éthyle. Les phases organiques réunies sont séchées sur sulfate de magnésium puis concentrées à sec sous pression réduite (2,7 kPa) à 30°C..On obtient ainsi, 0,9 g d'(amino-2 N-phényl-acétamido)-2 N-méthyl N-phényl-acétamide sous forme d'une huile utilisée telle quelle dans les synthèses ultérieures.

Le N-méthyl N-phényl (N-phényl phtalimido-2 acétamido)-2 acétamide peut être préparé de la manière suivante : à une suspension de 10,1 g d'acide (N-phényl phtalimido-2 acétamido)-2 acétique dans 125 cm³ de dichloro-1,2 éthane on ajoute 3,9 g de dichlorure d'oxalyle puis une goutte de diméthylformamide. le mélange est agité 2 heures à une température voisine de 25°C, puis on ajoute 7,7 g de N-méthyl aniline en solution dans 30 cm³ de dichloro-1,2 éthane. La solution obtenue est agitée pendant 2 heures à une température voisine de 25°C, puis lavée par 2 fois 80 cm³ d'eau distillée, séchée sur sulfate de magnésium et concentrée à sec sous pression réduite (2,7 kPa) à 40°C. On obtient, après recristallisation dans l'oxyde de diisopropyle, 9,6 g de N-méthyl N-phényl (N-phényl phtalimido-2 acétamido)-2 acétamide fondant à 216°C.

L'acide (N-phényl phtalimido-2 acétamido)-2 acétique peut être préparé de la manière suivante : à une solution de 8 g de (N-phényl phtalimido-2 acétamido)-2 acétate de tert-butyle dans 30 cm³ de dichlorométhane, on ajoute 17,9 g d'acide trifluoroacétique. La solution obtenue est agitée à reflux pendant 1 heure, puis concentrée à sec sous pression réduite (2,7 kPa) à 40°C. On obtient, après recristallisation dans l'éther diisopropylique, 5,9 g d'acide (N-phényl phtalimido-2 acétamido)-2 acétique fondant à 224°C.

Le (N-phényl phtalimido-2 acétamido)-2 acétate de tert-butyle peut être préparé de la manière suivante : à une solution de 207 g de N-phényl glycinate de tert-butyle dans 500 cm³ de dichloro-1,2 éthane, on ajoute 92,4 g d'hydrogénocarbonate de sodium. La suspension est agitée à une température voisine de 5°C et on ajoute une solution de 223 g de chlorure de phtalimido-2 acétyle dans 1100 cm³ de dichloro-1,2 éthane. Le mélange réactionnel est agité à reflux pendant 4 heures. Après séparation du produit insoluble par filtration, le filtrat est lavé par 300 cm³ d'eau distillée, séché sur sulfate de magnésium et concentré à sec sous pression réduite (2,7 kPa) à 40°C. On obtient, après recristallisation dans l'acétonitrile 236 g de (N-phényl phtalimido-2 acétamido)-2 acétate de tert-butyle fondant à 128°C.

Le N-phényl glycinate de tert-butyle peut être préparé de la manière suivante : à une solution de 56 g d'aniline dans 600 cm³ de dichloro-1,2 éthane, on ajoute 58 g de bromoacétate de tert-butyle et la solution obtenue est agitée à reflux pendant 48 heures. Après refroidissement, le produit insoluble est séparé par filtration et le filtrat est lavé par 200 cm³ d'une solution aqueuse d'acide chlorhydrique 0,1 N et par 3 fois 200 cm³ d'eau distillée. La phase organique est séchée sur sulfate de magnésium puis concentrée à sec sous pression réduite (2,7 kPa) à 40°C. On obtient ainsi 54 g de N-phényl glycinate de tert-butyle sous forme d'une huile utilisée telle quelle dans les synthèses ultérieures.

Le chlorure de phtalimido-2 acétyle peut être préparé selon la méthode décrite par W. GRASSMAN et E. SCHULTE-UEBLING, Chem. Ber., 83, 244 (1950).

Les médicaments selon l'invention sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles dans le traitement et la prévention des désordres liés à la CCK et à la gastrine au niveau du système nerveux et de l'appareil gastrointestinal. Ces composés peuvent donc être utilisés dans le traitement et la prévention des psychoses, des troubles anxieux, des attaques de panique, de la maladie de Parkinson, de la diskinésie tardive, du syndrome du colon irritable, de la pancréatite aiguë, des ulcères, des désordres de la motilité intestinale, de certaines tumeurs sensibles à la CCK, des troubles de la mémoire, comme analgésiques, comme potentialisateur de l'activité analgésique des médicaments analgésiques narcotiques et non narcotiques et comme régulateur de l'appétit, dans le sevrage aux traitements chroniques et abus d'alcool ou de médicaments et comme constricteur de la pupille de l'iris de l'oeil.

Les doses dépendent de l'effet recherché, de la durée du traitement et de la voie d'administration utilisée; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- {{[N-(méthoxy-3 phényl) N-(N-méthyl N-phényl-carbamoyméthyl) carbamoylméthyl]-3 uréido}-3 phényl}-1 éthanesulfonate de potassium-(RS) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle de comprimés dosés à 50 mg de produit actif ayant la composition suivante :
- (-)-{{[(N-(méthoxy-3 phényl) N-(N-méthyl N-phényl-carbamoylméthyl) carbamoylméthyl]-3 uréido}-3 phényl}-1 éthanesulfonate de potassium 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- {{[N-(N-méthyl N-phényl-carbamoylméthyl) N-phényl-carbamoylméthyl]-3 uréido}-3 phényl}-1 éthanesulfonate de potassium-(RS) 10 mg
- Acide benzoïque 80 mg
- Alcool benzylique 0,06 cm³
- Benzoate de sodium 80 mg
- Ethanol à 95 % 0,4 cm³
- Hydroxyde de sodium 24 mg
- Propylène glycol 1,6 cm³
- Eau q.s.p. 4 cm3

## Revendications

1. Composés de formule : 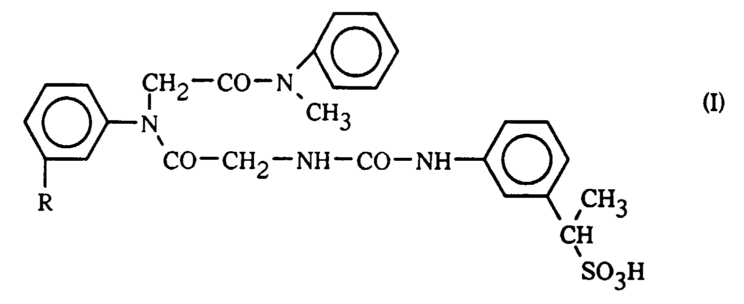 dans laquelle R représenté un atome d'hydrogène ou un radical méthoxy, leurs sels, leurs racémiques et leurs énantiomères.

2. Les composés suivants :
- acide {{[N-(méthoxy-3 phényl) N-(N-méthyl N-phényl-carbamoylméthyl) carbamoylméthyl]-3 uréido}-3 phényl}-1 éthanesulfonique-(RS)
- acide (-)-{{[N-(méthoxy-3 phényl) N-(N-méthyl N-phényl-carbamoylméthyl) carbamoylméthyl]-3 uréido}-3 phényl}-1 éthanesulfonique
- acide {{[N-(N-méthyl N-phényl-carbamoylméthyl) N-phényl-carbamoylméthyl]-3 uréido}-3 phényl}-1 éthanesulfonique-(RS).
et leurs sels

3. Procédé de préparation des composés de formule (I) caractérisé en ce que l'on fait réagir un dérivé de formule : 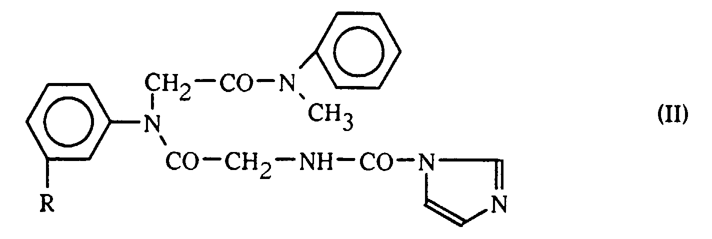 dans laquelle R représente un atome d'hydrogène ou un radical méthoxy, sur une amine de formule : 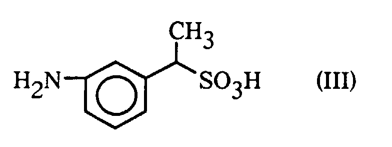 sous forme de sel et éventuellement sous forme d'un énantiomère, isole le produit et le transforme éventuellement en sel et/ou sépare les énantiomères.

4. Médicaments comprenant en tant que principe actif au moins un composé de formule (I) selon la revendication 1.

5. Médicaments selon la revendication 4 inhibiteurs de la CCK et de la gastrine.

## Patentansprüche

1. Verbindungen der Formel: 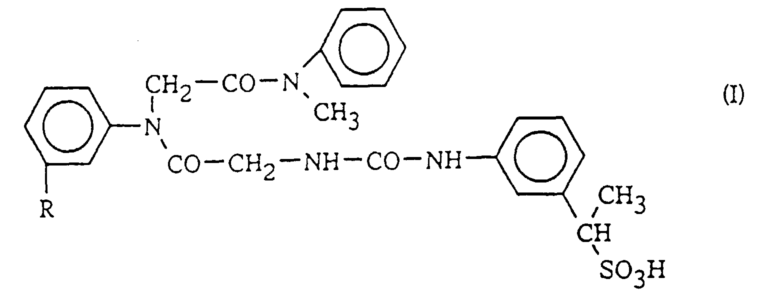 in welcher R für ein Wasserstoffatom oder einen Methoxyrest steht, deren Salze, deren Racemate und deren Enantiomere.

2. Die folgenden Verbindungen:
- (RS)-1-{3-{3-[N-(3-Methoxyphenyl)-N-(N-methyl-N-phenylcarbamoylmethyl)carbamoylmethyl]-ureido}phenyl}-ethansulfonsäure
- (-)-1-{3-{3-[N-(3-Methoxyphenyl)-N-(N-methyl-N-phenylcarbamoylmethyl)carbamoylmethyl]-ureido}-phenyl}-ethansulfonsäure
- (RS)-1-{3-{3-[N-(N-Methyl-N-phenylcarbamoylmethyl)-N-phenylcarbamoylmethyl]-ureido}-phenyl}-ethansulfonsäure
und deren Salze.

3. Verfahren zur Herstellung der Verbindungen der Formel (I), dadurch gekennzeichnet, daß man ein Derivat der Formel: 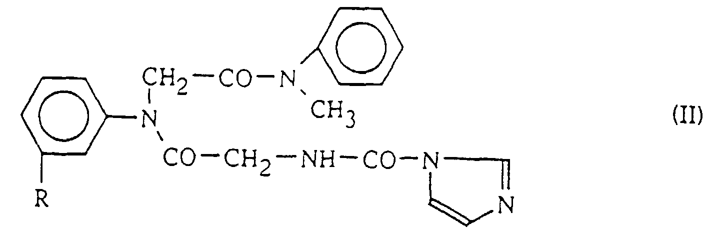 in welcher R für ein Wasserstoffatom oder einen Methoxyrest steht, mit einem Amin der Formel: 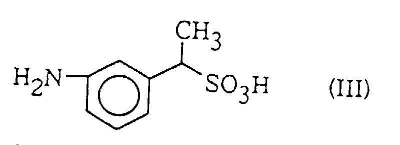 in Salz-Form und gegebenenfalls in Form eines Enantiomeren reagieren läßt, das Produkt isoliert und dieses gegebenenfalls in ein Salz überführt und/oder die Enantiomeren trennt.

4. Medikamente, die als Wirkstoff mindestens eine Verbindung der Formel (I) gemäß Anspruch 1 umfassen.

5. Medikamente nach Anspruch 4, die CCK und Gastrin inhibieren.

## Claims

1. Compounds of formula: 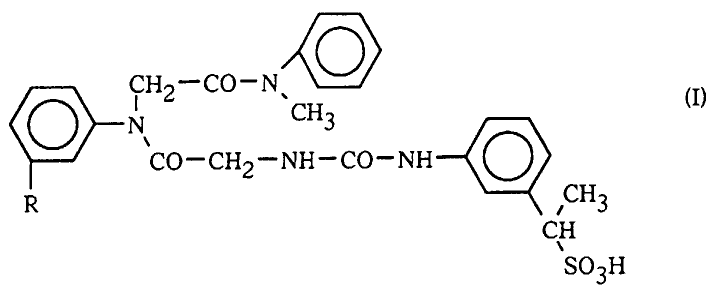 in which R represents a hydrogen atom or a methoxy radical, their salts, their racemates and their enantiomers.

2. The following compounds:
- (RS)-1-{3-{3-[N-(3-Methoxyphenyl)-N-(N-methyl-N-phenylcarbamoylmethyl)carbamoylmethyl]ureido}-phenyl}ethanesulphonic acid
- (-)-1-{3-{3-[N-(3-Methoxyphenyl)-N-(N-methyl-N-phenylcarbamoylmethyl)carbamoylmethyl]ureido}-phenyl}ethanesulphonic acid
- (RS)-1-{3-{3-[N-(N-Methyl-N-phenylcarbamoylmethyl)-N-phenylcarbamoylmethyl]ureido}phenyl}-ethanesulphonic acid
and their salts.

3. Process for preparing the compounds of formula (I), characterised in that a derivative of formula: 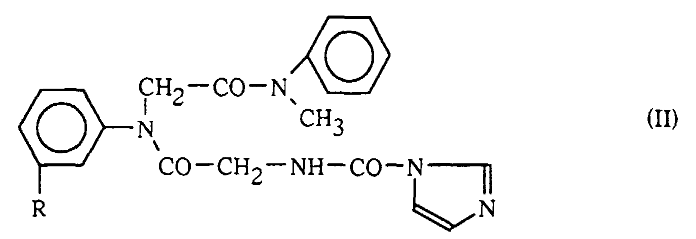 in which R represents a hydrogen atom or a methoxy radical, is reacted with an amine of formula: 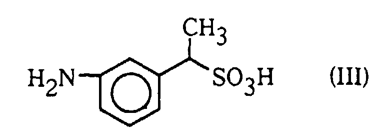 in the form of a salt and optionally in the form of an enantiomer, the product is isolated and is optionally converted to a salt and/or the enantiomers are separated.

4. Medicaments comprising, as active principle, at least one compound of formula (I) according to claim 1.

5. Medicaments according to claim 4 which are inhibitors of CCK and of gastrin.
